# DEMANDE DE BREVET EUROPEEN

(11) **EP 0 841 565 A1**
(43) Date de publication de la demande: **13.05.1998**
(21) Numéro de dépôt: 97402669.2
(22) Date de dépôt: 07.11.1997
(51) Int. Cl.: G01N 33/50, G01N 33/52

(54) **Procédé de détermination de la présence de pectines dans une solution aqueuse, notamment du type jus de fruits, moût de raisin, liquide alimentaire ou autre**

(30) Priorité: 08.11.1996 FR 9613642
(71) Demandeur: Pascal Biotech, 75017 Paris (FR)
(72) Inventeur: Belarbi, Abdel, 51430 Dezannes (FR); Gainvors, Angélique, 51140 Muizon (FR)
(74) Mandataire: Cournarie, Michèle

(57) **Abrégé**

L'invention concerne un procédé de détermination de la présence de pectines dans une solution aqueuse, caractérisé en ce que :
a) un échantillon de ladite solution aqueuse est mélangé avec de l'acétone dans un rapport en volume allant de 1:10 à 10:1,
b) le nombre de phases du mélange obtenu est ensuite déterminé. Un colorant spécifique des pectines peut être ajouté audit mélange afin de faciliter l'interprétation du résultat. Le procédé selon l'invention est plus particulièrement destiné à la détermination de la présence de pectines dans des jus de fruits, des moûts de raisins et autres liquides alimentaires, avant ou après traitement par des pectinases.

Il peut également être appliqué au criblage de micro-organismes à activité pectolytique.

## Description

La présente invention concerne un procédé de détermination de la présence de pectines dans une solution aqueuse, notamment du type jus de fruits, moût de raisin, liquide alimentaire ou autre, destiné à être utilisé, principalement, dans le domaine de l'agro-alimentaire, et plus particulièrement, lors de la fabrication de jus de fruits, ou de boissons alcoolisées.

En effet, les fruits comportent des pectines dans leurs parois cellulaires et dans leurs ciments intercellulaires (encore appelés lamelles moyennes). La quantité de pectine dépend, en particulier, de la maturation du fruit mais aussi de l'espèce végétale auquel il appartient.

Ces pectines sont des macromolécules glucidiques formées principalement de motifs composés d'acide D-galacturonique estérifié ou non par du méthanol et reliés entre eux par des liaisons α (1-4).

On les retrouve naturellement dans les jus de fruits, dont elles déterminent la viscosité et l'apparence. Plus la concentration en pectines est élevée, plus le jus de fruit est trouble et visqueux.

Cette viscosité élevée entraîne de nombreux problèmes pendant la fabrication et le conditionnement des jus de fruits, tandis que leur apparence trouble ne correspond pas aux goûts du consommateur.

Afin de remédier à ces inconvénients et donc, notamment, de clarifier les jus de fruits, les industriels de l'agro-alimentaire y ajoutent des pectinases, qui sont des enzymes pectolytiques qui vont déméthoxyler la pectine (pectine estérase) et couper la chaîne glucidique au niveau des liaisons α (1-4) de 2 acides galacturoniques non méthylés, (polygalacturonase) ou au niveau des liaisons α (1-4) entre 2 acides galacturoniques méthylés (pectine lyase), réalisant ainsi une dépolymérisation.

Mais ce traitement par pectinases est effectué de façon quasi-systématique alors qu'il ne se justifie pas toujours pour de faibles concentrations en pectines.

Les pectinases peuvent être ajoutées à différents stades de l'élaboration des jus de fruits, voire même juste avant leur conditionnement, mais le succès de leur activité de dégradation des pectines n'est visible qu'après plusieurs heures.

Ainsi l'insuccès éventuel du traitement par pectinases, qui se traduit par une absence de clarification, ne peut être constaté que tardivement.

Afin de parvenir à une gestion rationnelle des procédés d'élaboration des jus de fruits, différents procédés ont été mis au point afin d'évaluer, d'une part, la nécessité d'effectuer un traitement par pectinases et d'autre part, l'efficacité réelle d'un tel traitement.

Lorsqu'il s'agit de déterminer si un traitement par pectinases est nécessaire, les procédés reposent sur la détection de la présence de pectines dans le moût par précipitation à l'éthanol en milieu acide. Mais, cette méthode donne des résultats difficiles à interpréter et n'est utilisable que pour des concentrations en pectines supérieures à 300 mg/l.

D'autre part, lorsqu'il s'agit d'estimer la probabilité de réussite finale d'un traitement par pectinases, les procédés reposent sur la détection de l'activité des enzymes pectolytiques, par des méthodes chromatographiques notamment. Ces méthodes sont lourdes à mettre en oeuvre et trop lentes à fournir des résultats pour pouvoir rectifier le traitement de façon rapide.

En outre, elles ne renseignent pas directement sur l'efficacité réelle du traitement et ne donnent donc aucune garantie véritable quant à son succès final.

Ces deux types de procédés ne remplissent pas les attentes des utilisateurs, pour lesquels ils ne constituent pas une véritable aide à la décision en matière de traitement par des pectinases.

La présente invention a donc essentiellement pour but de remédier aux inconvénients susmentionnés des procédés de détermination de la présence de pectines dans une solution aqueuse, en fournissant un procédé qui soit à la fois plus sensible, plus rapide et plus simple à mettre en oeuvre.

Dans le cadre de l'invention, on entend par solution aqueuse toute solution contenant de l'eau comme constituant majoritaire ou minoritaire et pouvant également contenir toutes sortes de substances telles que des alcools, par exemple, ou bien encore des substances solides en suspension. Ceci recouvre, notamment, à titre d'exemple non limitatif, les jus de fruits, les moûts de raisin et les liquides alimentaires.

Le procédé de détermination de la présence de pectines dans une solution aqueuse est caractérisé par la réalisation des étapes suivantes :
a) un échantillon de ladite solution aqueuse est mélangé avec de l'acétone dans un rapport en volume allant de 1:10 à 10:1,
b) le nombre de phases du mélange obtenu est ensuite déterminé.

Le procédé selon l'invention remplit les objectifs susmentionnés grâce à l'utilisation d'un principe nouveau. Il s'agit d'exploiter la propriété qu'ont les pectines de se transformer en gel au contact de l'acétone.

Pour cela, on prélève un échantillon de la solution à analyser susceptible de contenir des pectines et on y ajoute de l'acétone.

Lorsque cette solution contient effectivement des pectines, on observe alors la formation instantanée d'un gel, qui remonte plus ou moins rapidement à la surface, pour former une couche qui surnage au-dessus du reste de ladite solution. Plus la concentration en pectines contenues dans la solution est élevée, plus le gel remonte rapidement à la surface, sans que cela nécessite plus de 10 minutes au maximum.

Le récipient dans lequel on a effectué le mélange contient alors deux phases distinctes, non-miscibles.

Par contre, lorsque la solution à analyser ne contient pas de pectines, l'ajout d'acétone ne provoque pas la formation d'une deuxième phase, et la solution reste homogène.

Le nombre de phases résultant du mélange de la solution à analyser avec de l'acétone permet donc de savoir si cette solution contient ou non des pectines, réalisant ainsi un test qualitatif.

Quant aux volumes respectifs d'acétone et de solution à analyser à mélanger, ils sont, de préférence, dans un rapport allant de 1:10 à 10:1.

En effet, lorsqu'on mélange plus de 10 volumes d'acétone pour 1 volume de solution à analyser, la hauteur de l'éventuelle phase gélifiée dans le récipient de test est alors faible par rapport à la hauteur de la phase contenant de l'acétone.

La lecture du test devient donc difficile, pouvant même mener à de mauvaises interprétations si la concentration en pectines est faible.

A contrario, lorsqu'on mélange plus de 10 volumes de solution à analyser pour 1 volume d'acétone, les pectines éventuelles pourraient ne pas être prises en gel intégralement, risquant là-aussi de fausser l'interprétation du résultat.

A l'intérieur de cette gamme, le rapport en volume à utiliser dépend principalement de la concentration en pectines de la solution aqueuse.

Dans le domaine de l'agro-alimentaire, par exemple, l'utilisateur va employer le procédé selon l'invention pour tester des solutions ayant des concentrations en pectines relativement voisines d'un lot de fabrication à l'autre.

Ainsi, il pourra avantageusement fixer un rapport des volumes respectifs d'acétone et de solution à analyser, qu'il utilisera de façon systématique. Cela lui permettra d'interpréter rapidement les résultats et de les comparer physiquement avec d'autres résultats obtenus précédemment, ceux-ci étant stables pendant plusieurs jours.

A titre d'exemple non limitatif, on peut mentionner que, pour un jus de raisin ou de pomme, en cours de fabrication, le rapport en volume optimal est de 1:1.

La limite de détection du procédé selon l'invention a été déterminée par une série d'essais utilisant des solutions contenant des concentrations décroissantes en poly(acide galacturonique), qui joue le rôle de pectine non estérifiée.

On constate l'apparition de deux phases bien distinctes jusqu'à une concentration en poly(acide galacturonique) égale à 50 mg/l, qui constitue donc la limite de détection du procédé selon l'invention.

Dans un mode de réalisation préféré de l'invention, on ajoute un colorant spécifique des pectines au mélange constitué d'un échantillon de la solution à analyser et d'acétone, préalablement à la détermination du nombre de phases dudit mélange, l'addition des différents constituants se faisant dans un ordre quelconque.

Ce colorant spécifique des pectines permet de faciliter la lecture rapide du résultat de l'application du procédé.

En effet, lorsque la solution à analyser ne contient pas de pectines, le colorant est réparti uniformément dans tout le mélange, mettant ainsi bien en évidence la présence d'une phase unique.

Par contre, lorsque la solution à analyser contient des pectines en quantité supérieure à 50 mg/l, le colorant est entièrement concentré dans la phase gélifiée constituée de pectines, laissant la seconde phase non-colorée, ce qui permet là aussi la mise en évidence claire de la présence de deux phases.

L'adjonction d'un colorant spécifique des pectines permet certes une interprétation visuelle rapide du résultat de l'application du procédé, mais elle permet également de pouvoir en réaliser une interprétation par colorimétrie, en déterminant, par exemple, la couleur de la phase inférieure du mélange.

Comme colorant spécifique des pectines, on préfère particulièrement le rouge de ruthénium, utilisé en solution aqueuse à 0,1 %.

Pour un mélange d'acétone et de solution à analyser ayant un volume total de 10 ml, on ajoute, de préférence, 1 ml de solution aqueuse de rouge de ruthénium à 0,1 %.

Selon le type de solution à laquelle on applique le procédé, il peut s'avérer nécessaire de tester au préalable les différentes substances susceptibles d'être présentes en même temps que les pectines afin de vérifier qu'elles ne prennent pas en gel.

Afin de vérifier la spécificité du procédé dans le domaine de l'agro-alimentaire, qui est le domaine d'utilisation préféré du procédé selon l'invention, les différents constituants susceptibles d'être présents dans des jus de fruits ont été testés en l'absence de pectines.

Ces essais ont porté sur les protéines, les sucres et les anthocyanes présents dans les jus de fruits et ont systématiquement fourni des mélanges finaux comportant une seule phase uniforme.

Dans le cas où des substances autres que des pectines prendraient en gel au contact de l'acétone, il serait alors indispensable d'ajouter au mélange initial un colorant spécifique des pectines tel que le rouge de ruthénium, pour lever l'incertitude.

Qu'il soit nécessaire ou non d'ajouter un colorant spécifique des pectines, on peut déterminer les quantités respectives des différentes phases du mélange, une fois la détermination du nombre de phase effectuée.

Cette étape supplémentaire n'intervient naturellement que lorsque la concentration en pectines est supérieure à 50 mg/l.

Elle permet de fournir une estimation de la concentration en pectines de la solution à analyser, car celle-ci est directement proportionnelle à la quantité de gel obtenu.

Cette quantification peut être faite en termes de volume, en utilisant, pour l'étape a) du procédé, par exemple, un récipient de mélange gradué. Cette méthode n'est pas très précise, mais présente l'avantage d'être rapide.

Si l'utilisateur souhaite des résultats plus précis, il peut, par exemple, filtrer le mélange biphasique sur un papier filtre, recueillant ainsi la phase gélifiée qu'il peut ensuite peser.

Afin de rendre la présente invention plus concrète, les exemples suivants viennent l'illustrer.

### EXEMPLE 1 : Application à un jus de pomme avant traitement par pectinases.

Un échantillon de 2 ml de jus de pomme fraîchement fabriqué est prélevé dans la cuve qui le contient et est versé dans un tube à essai.

On y ajoute ensuite 2 ml d'acétone et 0,2 ml d'une solution aqueuse de rouge de ruthénium à 0,1 %. Un gel coloré se forme immédiatement au sein du tube.

On laisse reposer le tube pendant 10 minutes. On observe alors qu'une phase gélifiée et colorée en rouge surnage au-dessus du reste de la solution, laissant la deuxième phase colorée en jaune clair, couleur naturelle du jus de pomme.

On en conclut donc que ce jus de pomme contient des pectines en concentration supérieure à 50 mg/l et qu'il est donc nécessaire d'effectuer un traitement par pectinases.

### EXEMPLE 2 : Application à un jus de pomme après traitement par pectinases

Le jus de pomme décrit dans l'exemple 1 fait l'objet d'un traitement par une pectinase « Endozym » (marque enregistrée) fabriquée par la société Pascal Biotech, à raison de 2 g par hl de jus de fruits.

Afin de vérifier rapidement l'efficacité du traitement, on prélève un échantillon de 2 ml du jus contenu dans la cuve.

De la même façon que dans l'exemple 1, on mélange, dans un tube à essai, l'échantillon de 2 ml avec 2 ml d'acétone et 0,2 ml de rouge de ruthénium à 0,1 %.

On obtient une solution monophasique homogène et uniformément colorée en rouge.

On laisse reposer le tube pendant 10 minutes. On n'observe aucun changement de l'apparence du contenu du tube à essai.

On peut donc conclure que le traitement par pectinases a été efficace et que la totalité des pectines présentes ont été dégradées.

On observe effectivement la clarification de ce jus de pomme, au bout de 5 heures.

### EXEMPLES 3 ET 4

Le protocole des exemples 1 et 2 est appliqué à un moût de raisin, avant et après traitement par des pectinases.

On observe des résultats similaires à ceux obtenus avec le jus de pomme.

Outre son application dans des procédés agro-alimentaires, le procédé selon l'invention peut également trouver, par exemple, une application dans le domaine de la microbiologie.

En effet, il peut être utilisé afin de déterminer rapidement si un micro-organisme produit ou non des enzymes pectolytiques.

De tels micro-organismes sont très recherchés car l'industrie est toujours intéressée par de nouvelles sources de production de pectinases.

La mise en application du procédé selon l'invention à ce type de détection nécessite quelques manipulations préalables.

Tout d'abord, le micro-organisme à tester est mis en culture dans un milieu adéquat, contenant 1 % de poly(acide galacturonique) non estérifié, par exemple, qui tient lieu de pectine.

Au bout de quelques jours de culture (temps en relation avec la physiologie du micro-organisme), on recueille le liquide surnageant, débarrassé des micro-organismes et on lui applique le procédé selon l'invention. Si on observe la formation de deux phases, on peut en conclure que le micro-organisme testé ne produit pas de polygalacturonases, car la pectine présente dans le milieu de culture n'a pas été dégradée.

Par contre, si on observe une seule phase homogène, on peut conclure que la pectine présente dans le milieu de culture a été dégradée et donc que le micro-organisme produit des enzymes polygalacturonases.

Lorsqu'on souhaite savoir si un micro-organisme produit d'autres enzymes pectolytiques, telle que des pectine-lyases, qui ne dégradent pas le même type de pectines que les polygalacturonases, il suffit d'injecter dans le milieu de culture des pectines adéquates à la place du poly(acide galacturonique) cité précédemment, en l'occurence des pectines méthylées.

Ainsi, pour détecter la production de pectine-lyases, par exemple, on utilisera une pectine estérifiée à plus de 75 % par du méthanol.

L'application du procédé selon l'invention à ce type de détection permet de faire un tri rapide parmi des centaines de micro-organismes, pour ne retenir que les plus intéressants.

Dans le domaine particulier de la clarification des jus de fruits, il permet avec un seul procédé de répondre à deux types de problèmes, qui étaient traités de façon distincte et approximative jusqu'à présent, à savoir la nécessité d'effectuer un traitement par pectinases et la vérification rapide de la réussite d'un tel traitement.

On voit donc que le procédé selon la présente invention permet de déterminer la présence ou l'absence de pectines dans toutes sortes de solutions aqueuses, éventuellement alcoolisées, susceptibles d'en contenir, mais aussi d'en estimer la concentration et cela de façon rapide, précise et simple à mettre en oeuvre.

Il va de soi que les différents modes de réalisation du procédé selon l'invention qui ont été décrits ci-dessus ont été donnés à titre d'exemples purement indicatifs et nullement limitatifs, et que de nombreuses modifications peuvent être facilement apportées par l'homme de l'art sans pour autant sortir du cadre de l'invention.

Ainsi, les méthodes d'interprétation des résultats de l'application du procédé, ainsi que les méthodes de quantification des concentrations en pectines peuvent être adaptées à loisir, en fonction des besoins et des impératifs des utilisateurs.

## Revendications

1. Procédé de détermination de la présence de pectines dans une solution aqueuse, caractérisé en ce que :
a) un échantillon de ladite solution aqueuse est mélangé avec de l'acétone dans un rapport en volume allant de 1:10 à 10:1,
b) le nombre de phases du mélange obtenu est ensuite déterminé.

2. Procédé de détermination de la présence de pectines dans une solution aqueuse selon la revendication 1, caractérisé en ce qu'un colorant spécifique des pectines est ajouté au mélange constitué d'un échantillon de ladite solution aqueuse et d'acétone, préalablement à la détermination du nombre de phases dudit mélange.

3. Procédé de détermination de la présence de pectines dans une solution aqueuse selon l'une ou l'autre des revendications 1 et 2, caractérisé en ce que la détermination du nombre de phases du mélange constitué d'un échantillon de ladite solution aqueuse, d'acétone et d'un éventuel colorant spécifique des pectines est effectuée de façon visuelle.

4. Procédé de détermination de la présence de pectines dans une solution aqueuse selon la revendication 2, caractérisé en ce que la détermination du nombre de phases du mélange constitué d'un échantillon de ladite solution aqueuse, d'acétone et de colorant spécifique des pectines est effectuée par colorimétrie.

5. Procédé de détermination de la présence de pectines dans une solution aqueuse selon l'une quelconque des revendications 2 à 4, caractérisé en ce que le colorant spécifique des pectines, qui est ajouté au mélange constitué d'un échantillon de ladite solution aqueuse et d'acétone, préalablement à la détermination du nombre de phases dudit mélange, est le rouge de ruthénium.

6. Procédé de détermination de la présence de pectines dans une solution aqueuse selon l'une quelconque des revendications 1 à 5, caractérisé en ce que les quantités respectives des phases du mélange constitué d'un échantillon de ladite solution aqueuse d'acétone et d'un éventuel colorant spécifique des pectines sont déterminées.

7. Application du procédé selon l'une quelconque des revendications 1 à 6, à la détermination de la présence de pectines dans des jus de fruits.

8. Application du procédé selon l'une quelconque des revendications 1 à 6, à la détermination de la présence de pectines dans des jus de fruits, à la suite d'un traitement par des enzymes pectolytiques.

9. Application du procédé selon l'une quelconque des revendications 1 à 6, à la mise en évidence d'une production d'enzymes pectolytiques par des micro-organismes.
